# EUROPÄISCHE PATENTSCHRIFT

(11) **EP 2 745 803 B1**
(45) Veröffentlichungstag und Bekanntmachung des Hinweises auf die Patenterteilung: **27.04.2016**
(21) Anmeldenummer: 12198313.4
(22) Anmeldetag: 20.12.2012
(51) Int. Cl.: A61C 19/00, A61L 2/07, A61L 2/16

(54) **Aufbereitungskammer oder Aufbereitungsträger sowie Vorrichtung zur Aufbereitung von zumindest einem medizinischen, insbesondere zahnärztlichen, Instrument**
Maintenance chamber or maintenance carrier and device for maintaining at least one medical, in particular dental instrument
Chambre de préparation ou support de préparation et dispositif de préparation d'au moins un instrument médical, en particulier dentaire

(43) Veröffentlichungstag der Anmeldung: 25.06.2014
(73) Patentinhaber: W & H Dentalwerk Bürmoos GmbH, 5111 Bürmoos (AT)
(72) Erfinder: WImmer, Stefan, 5121 Ostermiething (AT)

(56) Entgegenhaltungen:
- DE-U1-202012 002 701
- US-A1- 2004 001 783
- US-A1- 2004 126 274
- US-A1- 2011 206 555

## Beschreibung

Die vorliegende Erfindung bezieht sich auf eine Aufbereitungskammer oder auf einen Aufbereitungsträger zur Verwendung in einer Vorrichtung zur Aufbereitung von zumindest einem medizinischen, insbesondere zahnärztlichen, Instrument nach dem Oberbegriff des Anspruchs 1 sowie auf eine Aufbereitungsvorrichtung mit einer derartigen Aufbereitungskammer und/ oder einem derartigen Aufbereitungsträger nach Anspruch 11.

Aufbereitungsvorrichtung dienen zum Reinigen, Desinfizieren, Sterilisieren und/ oder Pflegen von medizinischen, insbesondere zahnärztlichen, Instrumenten. Als aufzubereitende Instrumente werden insbesondere gerade, gebogene oder pistolenförmige Handstücke, als auch Teile von Handstücken, z. B. Handstückköpfe mit einer Werkzeugaufnahme zur Aufnahme eines Behandlungswerkzeugs, Adapter und Kupplungen verstanden. Durch die Handstücke erstrecken sich oftmals Versorgungsleitungen zum Antrieb eines Behandlungswerkzeugs sowie Fluidleitungen. Bei den Leitungen handelt es sich insbesondere um Getriebekanäle oder Fluidkanäle für Luft, Wasser oder Spray.

Zur Aufbereitung der medizinischen Instrumente, insbesondere deren Antriebskanäle sowie Fluidkanäle, leiten die Vorrichtungen, insbesondere die Sterilisatoren, Autoklaven oder Thermodesinfektoren, zumindest ein Betriebsmedium in eine Aufbereitungskammer, in der das zumindest eine medizinische, insbesondere zahnärztliche, Instrument, vorzugsweise mittels eines Aufbereitungsträgers, aufgenommen ist, oder führen das Medium dem zumindest einen medizinischen Instrument direkt zu. Als Betriebsmedien können vorzugsweise Flüssigkeiten, wie zum Beispiel Heißwasser, oder Dämpfe, insbesondere gesättigter Wasserdampf, verwendet werden.

Im Stand der Technik ist es bekannt die Betriebsmedien in den Aufbereitungsvorrichtungen jeweils auf eine Betriebstemperatur zu erwärmen. Hierzu sind in den Aufbereitungsvorrichtungen Heizvorrichtungen angeordnet. Bei Sterilisatoren oder Autoklaven sind diese als eigenständige Verdampfer ausgebildet, welche das Betriebsmedium Wasser in gesättigten Wasserdampf umwandeln. Dieser Dampf wird anschließend mittels zumindest einer Leitung der Aufbereitungskammer, dem Aufbereitungsträger oder direkt dem medizinischen Instrument zugeführt, um das zumindest eine medizinische Instrument auf 134 Grad Celsius zu erwärmen.

Des Weiteren ist es im Stand der Technik, insbesondere bei Sterilisatoren und Autoklaven, bekannt die Aufbereitungskammern der Aufbereitungsvorrichtungen mittels weiterer Heizelemente, welche ebenfalls in den Aufbereitungsvorrichtungen angeordnet sind, vorzuwärmen. Hierdurch wird es ermöglicht die Menge an Dampf, welche benötigt wird um das zumindest eine medizinische Instrument auf 134 Grad Celsius zu erwärmen, zu reduzieren. Die Heizelemente sind hierbei derart in den Aufbereitungsvorrichtungen angeordnet, dass diese in thermischer Verbindung mit den Aufbereitungskammern stehen, wodurch ein Wärmeübertrag von den Heizelementen auf die Aufbereitungskammern erfolgt.

Eine derartige Vorrichtung ist insbesondere aus der US 2004/0001783 A1 bekannt.

Diese bekannte Vorrichtung zum Sterilisieren von verunreinigten medizinischen Instrumenten umfasst einen zylindrischen Behälter zur Aufnahme der zu sterilisierenden Instrumente. Der Behälter ist hierbei in dem Gehäuse der Aufbereitungsvorrichtung angeordnet. Der Behälter selbst ist doppelwandig ausgebildet. Zwischen der inneren und äußeren Wand des Behälters sind mehrere elektrische Heizelemente angeordnet. Diese werden mittels einer Trägerplatte, welche unterhalb des Behälters in der Vorrichtung angeordnet ist, zwischen der inneren und äußeren Wand des Behälters positioniert.

Die US 2004/0126274 A1 offenbart eine Vorrichtung zum Desinfizieren von Zahnbürsten.

Hierzu weist die Vorrichtung eine Kammer zur Aufnahme der Bürsten sowie eine Vorrichtung zum Erzeugen von Dampf auf. Die Vorrichtung zum Erzeugen von Dampf umfasst einen Heizwiderstand, insbesondere einen PTC-Thermistor, um elektrische Energie in Wärmeenergie umzuwandeln. Das Heizelement ist hierbei als eigenständiges Bauteil ausgebildet und mittels einer Halterung an der Unterseite der Kammer befestigt.

Weitere Vorrichtungen zum Aufbereiten von medizinischen Instrumenten sind beispielsweise aus der DE 20 2012 002 701 U1 und der US 2011/0206555 A1 bekannt.

Auch bei diesen bekannten Vorrichtungen ist das Heizelement als eigenständiges Bauteil, insbesondere als Heizschleife, in der Aufbereitungskammer für die medizinischen Instrumente oder außerhalb der Kammer in der Aufbereitungsvorrichtung angeordnet.

Als nachteilig dieser Ausgestaltungen der Aufbereitungsvorrichtungen erweist sich die Anordnung der mehreren, insbesondere eigenständigen, Heizvorrichtungen bzw. Heizelemente in den Aufbereitungsvorrichtungen.

Durch die Ausbildung der Heizvorrichtungen bzw. der Heizelemente als eigenständige Bauteile ist eine Übertragung der erzeugten Wärmemengen von den jeweiligen Heizvorrichtungen bzw. Heizelementen direkt oder mittels eines oder mehrerer Betriebsmedien zu den Aufbereitungskammern, Aufbereitungsträgern oder zu den medizinischen Instrumenten notwendig. Mit dieser Übertragung der erzeugten Wärmemengen sind jedoch Nachteile verbunden. Insbesondere führt der Transport der Betriebsmedien von den Heizvorrichtungen zu den Aufbereitungskammern mittels Fluid- oder Medienleitungen zu thermischen Verlusten. Des Weiteren stellt die Kopplung der eigenständigen Heizelemente mit den Aufbereitungskammern ein Problem dar. Um die thermischen Verluste bei der Übertragung der Wärmeenergien möglichst gering zu halten, ist es erforderlich, die Heizelemente an die Formen der Aufbereitungskammern anzupassen. Die Kammern selbst sind jedoch vorzugsweise an die Form der aufzubereitenden medizinischen Instrumente angepasst und weisen hierdurch zum Teil komplexe Formen auf, was die Kopplung der Heizelemente an die Aufbereitungskammern erschwert.

Einen weiteren Nachteil stellt der Platzbedarf für die mehreren Heizvorrichtungen bzw. Heizelemente sowie deren Verbindungsleitungen zu den Aufbereitungskammern in den Aufbereitungsvorrichtungen dar. Diese müssen zusätzlich zu den Aufbereitungskammern, der Steuervorrichtungen sowie weiterer relevanter Bauteile in den Aufbereitungsvorrichtungen aufgenommen werden. Dies wiederum steht einer anwenderfreundlichen Ausgestaltung der Aufbereitungsvorrichtungen, insbesondere einer möglichst platzsparenden Ausformung der Vorrichtungen, entgegen.

Ebenso stellen die hohen Kosten für die zahlreichen Bauteile, insbesondere zur Erwärmung der Betriebsmedien, zum thermisch isolierten Transport der Medien sowie zum Vorheizen der Aufbereitungskammern, einen Nachteil dar.

Der vorliegenden Erfindung liegt daher die Aufgabe zugrunde eine Aufbereitungskammer oder einen Aufbereitungsträger sowie eine Vorrichtung zur Aufbereitung von zumindest einem medizinischen, insbesondere zahnärztlichen, Instrument mit einer derartigen Aufbereitungskammer oder einem derartigen Aufbereitungsträger zu schaffen, welche bei einfacher Herstellung die Nachteile des Stand der Technik vermeiden und es insbesondere ermöglichen in platzsparender Weise Wärmeenergie in der Aufbereitungsvorrichtung zu Erzeugen und auf das zumindest eine aufzubereitende medizinische Instrument zu übertragen sowie thermischen Verluste in der Aufbereitungsvorrichtung zu vermeiden.

Zur Lösung dieser Aufgabe wird gemäß einem ersten Ausführungsbeispiel eine Aufbereitungskammer oder ein Aufbereitungsträger zur Verwendung in einer Vorrichtung zur Aufbereitung von zumindest einem medizinischen, insbesondere zahnärztlichen, Instrument mit einem Gehäuse, welches das zumindest eine medizinische Instrument zumindest teilweise umgibt, vorgeschlagen, wobei das Gehäuse zumindest eine Leiterbahn aus elektrisch leitendem Material zum Umwandeln elektrischer Energie in Wärmeenergie und zumindest zwei elektrische Kontakte aufweist, um die zumindest eine Leiterbahn mit elektrischer Energie zu versorgen und wobei die elektrische leiterbahn durch eine ditcht auf der Aufsereitungschanner oder dem träger aufgesrachte, insbesondere als Dünnschicht oder Dichschicht, ausgebildete beschintung gekennzeichnet ist. Gemäß einem zweiten Ausführungsbeispiel weist die zumindest eine Leiterbahn eine Trägerschicht und/ oder Deckschicht aus dielektrischem Material auf, um die Leiterbahn elektrisch zu isolieren. Insbesondere bei der Verwendung der Aufbereitungskammer oder des Aufbereitungsträgers in einer Vorrichtung zur Sterilisation der medizinischen Instrumente ist die Aufbereitungskammer und/ oder der Träger aus elektrisch leitendem Material, insbesondere aus Edelstahl gefertigt. Um nun die zumindest eine Leiterbahn direkt auf der Kammer, insbesondere an deren Innen- oder Außenseite, oder dem Träger elektrisch isoliert zu positionieren, wird erfindungsgemäß auf der Kammer oder dem Träger eine Trägerschicht angebracht. Die Deckschicht, welche ebenfalls ein dielektrisches Material aufweist, dient zum elektrischen Isolieren der zumindest einen Leiterbahn gegenüber dem zumindest einen aufzubereitenden medizinischen Instrument und/ oder weiterer, insbesondere elektrisch leitender, Bauteile in der Aufbereitungsvorrichtung, welche insbesondere benachbart zur der Aufbereitungskammer oder dem Träger in der Vorrichtung angeordnet sind.

Die zumindest auch die Trägerschicht und die Deckschicht, sind vorzugsweise jeweils durch eine Dünnschicht oder eine Dickschicht ausgebildet. Diese Schichten werden bevorzugt durch ein Beschichtungsverfahren, insbesondere durch ein Dünnfilm- oder Dickfilmverfahren, direkt auf der Aufbereitungskammer oder dem Aufbereitungsträger aufgebracht.

Gemäß einem weiteren Ausführungsbeispiel weist die zumindest eine Leiterbahn mehrere Abschnitte auf, welche in unterschiedlichen Abständen zueinander an dem Gehäuse der Aufbereitungskammer oder des Aufbereitungsträgers angeordnet sind. Hierdurch ist es möglich die erzeugte Wärmemenge pro Fläche an dem Gehäuse zu variieren, wodurch eine homogene Temperaturverteilung in der Kammer oder dem Träger erzielt werden kann. Das Gehäuse der Aufbereitungskammer oder des Trägers mit der zumindest einen Leiterbahn ist vorzugsweise in zwei Teilbereiche geteilt, wobei ein erster Teilbereich in vertikaler Sicht unterhalb eines zweiten Bereichs liegt. Um nun eine homogene Temperaturverteilung in der Kammer zu erzeugen, weisen die mehreren Abschnitte der zumindest einen Leiterbahn in dem unteren Teilbereich kleinere Abstände auf, als im oberen Teilbereich.

Gemäß einem weiteren Ausführungsbeispiel ist die zumindest eine Leiterbahn derart an dem Gehäuse der Aufbereitungskammer oder des Aufbereitungsträgers angeordnet, dass diese das zumindest eine medizinische Instrument zumindest an zwei Seiten umgibt. Bevorzugt erstreckt sich die Leiterbahn hierbei wendelförmig um das zumindest eine medizinische Instrument und entlang dessen Längsachse, insbesondere in axialer und radialer Richtung. Des Weiteren ist bevorzugt eine zweite, insbesondere spiralförmige oder schneckenförmige, Leiterbahn an einer weiteren Seite des Gehäuses angeordnet. Diese ist bevorzugt separat mit elektrischer Energie versorgbar und somit ansteuerbar.

Gemäß einem weiteren Ausführungsbeispiel sind die zumindest zwei Leiterbahnen derart an dem Gehäuse angeordnet, dass zumindest eine der zwei Leiterbahnen zwischen der jeweils anderen Leiterbahn angeordnet ist. Die Leiterbahnen greifen somit ineinander.

Gemäß einem weiteren Ausführungsbeispiel weist das Gehäuse der Aufbereitungskammer oder des Aufbereitungsträgers zumindest eine Medienzuführung zum Einbringen zumindest eines Betriebsmediums in die Aufbereitungskammer oder in den Aufbereitungsträger auf. Diese ist vorzugsweise als Düse ausgebildet. Das zugeführte Betriebsmedium wird hierbei bevorzugt auf die, insbesondere spiralförmig ausgebildete, zweite Leiterbahn geleitet, um das Medium direkt in der Kammer oder dem Träger zu erwärmen. Die zweite Leiterbahn dient somit zusätzlich als Heizelement zur Erwärmung bzw. Verdampfung des Betriebsmediums. Neben der Medienzuführung weist die Aufbereitungskammer oder der Aufbereitungsträger bevorzugt zumindest einen Medienablauf auf, um die Betriebsmittel aus der Kammer oder dem Träger zu leiten.

Gemäß einem weiteren Ausführungsbeispiel weist das Gehäuse der Aufbereitungskammer oder des Aufbereitungsträgers zumindest eine Anschlussvorrichtung zur Aufnahme des zumindest einen medizinischen Instruments auf. Die Anschlussvorrichtung selbst kann gleichzeitig als Medienzuführung ausgebildet sein. Hierbei umfasst diese bevorzugt eine Verbindungskupplung zum Anschluss des zumindest einen medizinischen Instruments, insbesondere eines Hand- oder Winkelstücks.

Gemäß einem weiteren Ausführungsbeispiel ist das Gehäuse der Aufbereitungskammer oder des Aufbereitungsträgers durch zumindest ein erstes und zweites Gehäuseteil gebildet, welche zum Einbringen und Entnehmen des zumindest einen medizinischen, insbesondere zahnärztlichen, Instruments, relativ zueinander drehbar oder schiebbar miteinander verbunden sind. Hierbei weist das Gehäuse vorzugsweise zwei Leiterbahnen zum Umwandeln elektrischer Energie in Wärmeenergie auf, wobei jeweils eine Leiterbahn an dem ersten und zweiten Gehäuseteil angeordnet ist.

Gemäß eines ersten Ausführungsbeispiel der Vorrichtung zur Aufbereitung von zumindest einem medizinischen, insbesondere zahnärztlichen, Instrument umfasst diese eine Steuervorrichtung zum Steuern und/ oder Regeln eines Aufbereitungsprozesses, eine Energieversorgung zum Anschluss der Aufbereitungsvorrichtung, insbesondere der Steuervorrichtung, an eine Energiequelle, zumindest eine Medienversorgung mit zumindest einem Medienspeicher und/ oder zumindest einem Anschluss für eine externe Medienquelle sowie eine Aufbereitungskammer und/ oder einen Aufbereitungsträger für das zumindest eine medizinische, insbesondere zahnärztliche, Instrument, wobei die Aufbereitungskammer und/ oder der Aufbereitungsträger gemäß einem der oben ausgeführten Ausführungsbeispiele ausgebildet sind.

Gemäß einem zweiten Ausführungsbeispiel der Vorrichtung zur Aufbereitung von zumindest einem medizinischen, insbesondere zahnärztlichen, Instrument weist die Aufbereitungsvorrichtung eine Tür auf, wobei die Steuervorrichtung, die Energieversorgung oder die Medienversorgung und zumindest eines der beiden Gehäuseteile der Aufbereitungskammer oder des Aufbereitungsträgers in oder an der Tür der Aufbereitungskammer angeordnet sind. Durch diese Anordnung der für den Aufbereitungsprozess notwendigen Bauteile sowie der zumindest einen Leiterbahn in bzw. an der Tür der Aufbereitungsvorrichtung ist es möglich, dieses als Standardbauteil auszubilden und für unterschiedlich ausgebildete Aufbereitungsvorrichtungen zu verwenden. Insbesondere unterscheiden sich diese Vorrichtungen zur Aufbereitung hinsichtlich der Größe des Aufbereitungsraumes.

Gemäß einem weiteren Ausführungsbeispiel der Aufbereitungsvorrichtung weist die Steuervorrichtung eine Schaltung zur Identifikation des zumindest einen in der Aufbereitungskammer oder in dem Aufbereitungsträger aufgenommenen Instruments auf. Hierzu ist die zumindest eine, vorzugsweise spulenförmige, Leiterbahn zum Umwandeln elektrischer Energie in Wärmeenergie, welche an dem Gehäuse der Aufbereitungskammer oder des Aufbereitungsträgers angeordnet ist, mit der Steuervorrichtung verbunden und zum Senden und/ oder Empfangen von Daten und/ oder Energie ausgebildet. Daten und/ oder Energie können somit zwischen dem Instrument, insbesondere zwischen einer Speichereinheit in oder an dem medizinischen Instrument, und der Aufbereitungsvorrichtung übertragen werden, um so automatisch, basierend auf den übermittelten Daten, den Aufbereitungsprozess für das medizinische Instrument auszuwählen.

Gemäß einem weiteren Ausführungsbeispiel der Aufbereitungsvorrichtung weist die Steuervorrichtung einen Schaltkreis zur Erzeugung von Wirbelströmen in der Aufbereitungskammer oder in dem Aufbereitungsträger auf. Dazu ist die zumindest eine Leiterbahn zum Umwandeln elektrischer Energie in Wärmeenergie an dem Gehäuse der Aufbereitungskammer oder an dem Aufbereitungsträger zum Erzeugen von magnetischen Wechselfeldern ausgebildet.

Die vorliegende Aufbereitungskammer, der Aufbereitungsträger sowie die Aufbereitungsvorrichtung zeichnen sich durch eine Reihe erheblicher Vorteile aus.

Einen Vorteil der Erfindung bildet die Möglichkeit die benötigte Wärmeenergie direkt an bzw. durch die Aufbereitungskammer oder den Aufbereitungsträger selbst zu erzeugen. Eine Übertragung der erzeugten Wärmemengen von einer separaten und eigenständigen Heizvorrichtungen bzw. einem Heizelement zu der Aufbereitungskammer ist nicht mehr notwendig. Hierdurch entfallen die damit verbunden thermischen Verluste bei der Übertragung der Wärmeenergien.

Einen weiteren Vorteil der Erfindung bildet die Möglichkeit die Aufbereitungskammer oder den Aufbereitungsträger unabhängig von einem eigenständigen Heizelement auszubilden. Durch die Anordnung der Leiterbahn zum Umwandeln elektrischer Energie in Wärmeenergie, direkt an der Kammer oder dem Träger wird es ermöglicht die Form des Gehäuses beliebig auszubilden, insbesondere an die Form des aufzubereitenden medizinischen Instruments anzupassen.

Des Weiteren stellt die Ausbildung der zumindest einen Leiterbahn durch ein Beschichtungsverfahren, insbesondere durch ein Dünnfilm- oder Dickfilmverfahren, einen Vorteil dar. Die Ausbildung der zumindest einen Leiterbahn als Dünnschicht oder Dickschicht ermöglicht, auf Grund der geringen Masse der Schicht, ein schnelles Aufheizen der Leiterbahn und somit der Aufbereitungskammer oder des Aufbereitungsträgers.

Einen weiteren Vorteil der Erfindung bildet die Möglichkeit der anwenderfreundlichen Ausgestaltung der Aufbereitungsvorrichtungen. Durch die Ausbildung der erfindungsgemäßen Aufbereitungskammer oder des Aufbereitungsträgers mit einer Leiterbahn aus elektrisch leitendem Material zum Umwandeln elektrischer Energie in Wärmeenergie ist es möglich die Wärmeenergie direkt an der Aufbereitungskammer zu erzeugen. Separat angeordnete Heizvorrichtungen bzw. Heizelemente sowie Verbindungsleitungen werden nicht mehr benötigt. Dies ermöglicht eine Optimierung der Baugröße der Aufbereitungsvorrichtung, insbesondere dessen Gehäuses.

Die Anbringung einer zusätzlichen zweiten oder dritten Leiterbahn, insbesondere einer Ersatzleiterbahn, welche erst bei Defekt einer ersten oder zweiten Leiterbahn in Betrieb genommen wird, ermöglicht des Weiteren ein einfaches und leichtes Instandsetzen eines defekten Heizelements der Aufbereitungsvorrichtung.

Nachfolgend wird die Erfindung anhand mehrerer Ausführungsbeispiele und in Verbindung mit den beigefügten Zeichnungen erläutert.

Dabei zeigt:
Figur 1 eine perspektivische Darstellung eines ersten Ausführungsbeispiels einer Aufbereitungsvorrichtung zur Aufbereitung von zumindest einem medizinischen,
insbesondere zahnärztlichen, Instrument,
Figur 2 in einer schematischen Darstellung einen Schnitt durch die Aufbereitungsvorrichtung aus Figur 1,
Figur 2A ebenfalls in Schnittdarstellung einen Ausschnitt aus dem Gehäuse der Aufbereitungskammer aus Figur 2,
Figur 2B ebenfalls in Schnittdarstellung einen weiteren Ausschnitt aus dem Gehäuse der Aufbereitungskammer aus Figur 2,
Figur 3 eine perspektivische Darstellung eines Aufbereitungsträgers zur Aufnahme von zumindest einem medizinischen, insbesondere zahnärztlichen, Instrument in einer Aufbereitungsvorrichtung;

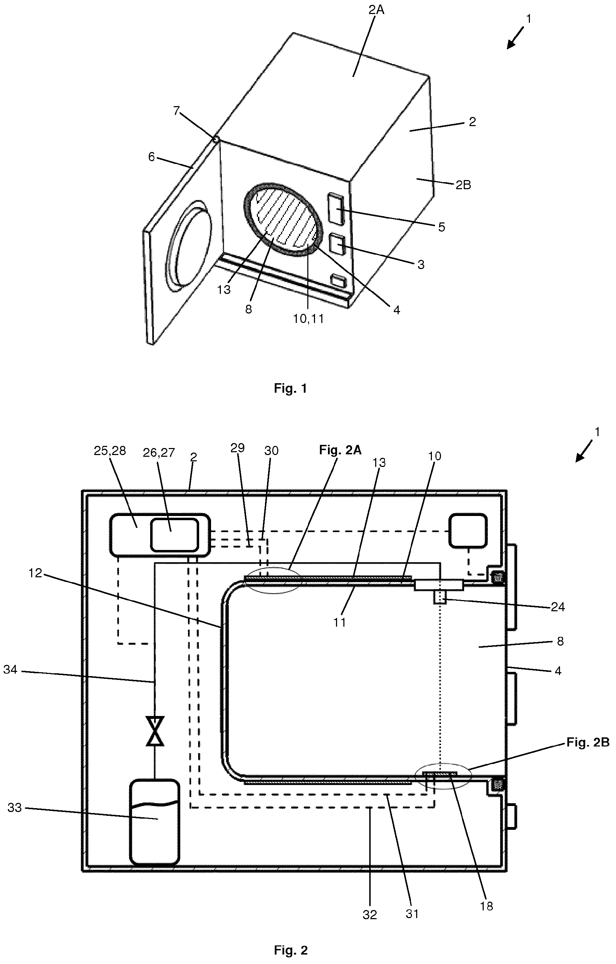

Die Figur 1 zeigt ein erstes Ausführungsbeispiel einer Aufbereitungsvorrichtung 1 zur Aufbereitung von zumindest einem medizinischen, insbesondere zahnärztlichen, Instrument. Diese ist vorzugsweise in Form eines Sterilisators 1, insbesondere eines Dampfsterilisators ausgebildet. Der Sterilisator 1 umfasst hierbei ein Gehäuse 2 mit mehreren Außenwänden 2A, 2B. Eine Außenwand, insbesondere eine Seitenwand, bildet vorzugsweise die Bedienungsseite des Reinigungs- oder Pflegegeräts 1. Diese weist mehrere Bedienelemente 3, die insbesondere zur Auswahl unterschiedlicher Betriebsprogramme oder zur Einstellung von Betriebsparametern dienen, auf. Eine Anzeige 5 zeigt die ausgewählten Betriebsprogramme oder Parameter des ausgewählten Aufbereitungsprozesses an. Neben den Bedienelementen 3 weist das Gehäuse 2 des Sterilisators 1 eine Öffnung 4 auf, die mit einer Aufbereitungskammer 8 des Sterilisators 1 verbunden ist. Durch die Öffnung 4 kann ein aufzubereitendes Instrument oder ein Aufbereitungsträger für das zumindest eine medizinische Instrument in die Aufbereitungskammer 8 eingebracht oder daraus entnommen werden. Die Öffnung 4 ist mittels einer Tür 6, welche bevorzugt drehbar über ein Scharnier 7 zu den mehreren Wänden 2A, 2B des Sterilisators 1 gelagert ist, verschließbar.

Die Aufbereitungskammer 8 selbst ist durch ein Gehäuse 10, welches bevorzugt aus Edestahl gefertigt ist, gebildet. Zumindest ein Teil des Gehäuses 10 ist vorzugsweise an der Tür 6 angeordnet, welches zum Einbringen und Entnehmen des zumindest einen medizinischen, insbesondere zahnärztlichen, Instruments, relativ zu dem Gehäuse 10 drehbar oder schiebbar gelagert ist. Bevorzugt an der Innenseite 11 des Gehäuses 10 ist zumindest eine erste Leiterbahn 13 aus elektrisch leitendem Material zum Umwandeln elektrischer Energie in Wärmeenergie angeordnet. Hierdurch ist es möglich Wärmeenergie direkt an bzw. durch die Aufbereitungskammer 8 zu erzeugen. Alternativ kann die Leiterbahn 13 auch an der Außenseite des Gehäuses 10 angebracht sein. Um die zumindest eine Leiterbahn 13 direkt auf der Kammer 8, insbesondere auf dem elektrisch leitenden Kammergehäuse 10, elektrisch isoliert zu positionieren, weist die Leiterbahn 13 eine Trägerschicht und/ oder Deckschicht aus dielektrischem Material auf. Die Leiterbahn 13 sowie die mehreren Schichten sind hierzu bevorzugt durch ein Dünnschicht- oder Dickschichtverfahren gebildet.

Die zumindest eine Leiterbahn 13 ist in diesem Ausführungsbeispiel derart an dem Gehäuse 10 der Aufbereitungskammer 8 angeordnet, dass diese die Aufbereitungskammer 8 zumindest an zwei Seiten umgibt. Hierzu erstreckt sich die Leiterbahn bevorzugt wendelförmig auf dem Gehäuse 10 der Kammer 8. Alternativ kann die Leiterbahn 13 in Form einer Helix ausgebildet sein, welche sich um die bevorzugt zylindrisch ausgebildete Aufbereitungskammer 8, insbesondere um deren Mantelfläche, windet.

Die Aufbereitungskammer 8 selbst ist bevorzugt lösbar von dem Sterilisator 1 ausgebildet.

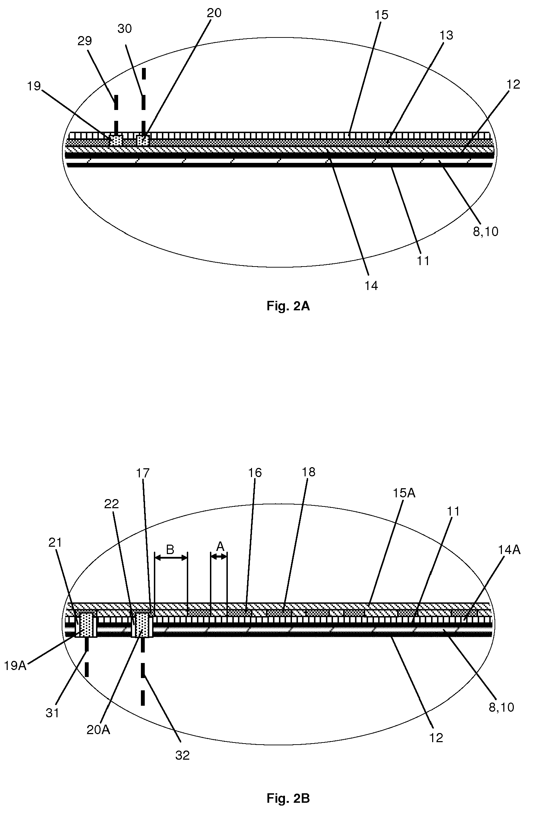

In Figur 2 ist in Schnittdarstellung das erste Ausführungsbeispiel der Aufbereitungsvorrichtung 1 aus Figur 1 in einer schematischen Darstellung gezeigt. Im Inneren des Gehäuses 2 des Sterilisators 1 sind neben der Aufbereitungskammer 8, welche über die Öffnung 4 zugänglich ist, zumindest eine Steuervorrichtung 25 sowie weitere für den Aufbereitungsprozess notwendige Bauteile, wie z.B. eine Energieversorgung 28 zum Anschluss des Sterilisators 1, insbesondere der Steuervorrichtung 25, an eine Energiequelle und eine Medienversorgung 33 angeordnet. Die Aufbereitungskammer 8 des Sterilisators ist bevorzugt als Druckkessel ausgeführt.

Die Medienversorgung 33 umfasst zumindest einen Medienspeicher für ein Arbeitsmedium oder einen Medienanschluss zum Anschließen des Sterilisators 1 an eine externe Medienquelle. Des Weiteren weist die Medienversorgung 33 insbesondere Mittel zum Transportieren des zumindest einen Aufbereitungsmediums in die Aufbereitungskammer 8, wie zum Beispiel Ventile, Pumpen oder Medienleitungen 34 auf. Hierzu sind die Medienleitungen 34 mit der Aufbereitungskammer 8, insbesondere über zumindest eine Medienzuführung 24, welche bevorzugt als Düse ausgebildet ist, verbunden. Des Weiteren weist die Aufbereitungskammer 8 zumindest einen Medienabfluss zum Ableiten der in die Kammer geleiteten Medien auf.

Die Steuervorrichtung 25 umfasst bevorzugt eine oder mehrere Steuerschaltungen, einen Mikrocontroller und Sensoren, insbesondere zumindest einen Temperatursensor in der Aufbereitungskammer 8, zur Detektion von Betriebsparametern. Des Weiteren ist die Steuervorrichtung 25 über Steuerleitungen mit der Medienversorgung 33, mit den Bedienelementen und der Anzeige verbunden. Für zumindest eine erste und zweite Leiterbahn 13 und 18 weist der der Sterilisator 1 zumindest zwei elektrische Zuführungsleitungen 29, 30 und 31, 32 auf, welche die Leiterbahnen 13 und 18 mit der Steuervorrichtung 25 und/ oder der Energieversorgung 28 elektrisch verbinden. Die Steuervorrichtung 25 steuert und/ oder regelt hierdurch den Betrieb der Aufbereitungsvorrichtung 1.

Gemäß einem zweiten Ausführungsbeispiel der Aufbereitungskammer 8, wie in Figur 2 gezeigt, weist das Gehäuse 10 der Aufbereitungskammer 8 eine erste Leiterbahn 13 an der Außenseite 12 und eine zweite Leiterbahn 18 an der Innenseite 11 des Gehäuses 10 auf. Beide Leiterbahnen 13, 18 sind bevorzugt separat über die Leitungen 29, 30 und 31, 32 mit elektrischer Energie versorgbar. Hierbei dient die erste Leiterbahn 13 insbesondere zum Vorwärmen der Aufbereitungskammer 8. Die zweite Leiterbahn 18 dient bevorzugt zur Erwärmung bzw. Verdampfung des Betriebsmediums. Hierzu wird das Betriebsmedium mittels der Medienzuführung 24 direkt auf die, insbesondere spiralförmig ausgebildete, zweite Leiterbahn 18 geleitet, um das Medium direkt in der Kammer 8 zu erwärmen bzw. zu verdampfen.

Die Steuervorrichtung 25 weist vorzugsweise des Weiteren eine Schaltung 26 zur Identifikation des zumindest einen in der Aufbereitungskammer 8 oder in dem Aufbereitungsträger 9 aufgenommenen Instruments auf. Hierzu ist die zumindest eine Leiterbahn 13 oder 18 an dem Gehäuse 10 der Aufbereitungskammer 8 zum Senden und/ oder Empfangen von Daten und/ oder Energie ausgebildet ist. Diese weist bevorzugt die Form einer Spule auf. Daten und/ oder Energie können somit zwischen dem Instrument, insbesondere zwischen einer Speichereinheit in oder an dem medizinischen Instrument, und der Aufbereitungsvorrichtung 1 übertragen werden. Bevorzugt ist die Schaltung 26 ausgebildet, das von dem Sterilisator 1 durchgeführte Aufbereitungsverfahren zu überwachen und nur dann Daten über das Verfahren mittels der zumindest einen Leiterbahn 13 oder 18 an das zumindest eine medizinische Instrument zu übertragen, wenn der Sterilisator 1 die Aufbereitung tatsächlich ausgeführt hat.

Des Weiteren weist die Steuervorrichtung 25 bevorzugt einen Schaltkreis 27 zur Erzeugung von Wirbelströmen in der Aufbereitungskammer 8 auf. Die zumindest eine Leiterbahn 13 oder 18 an dem Gehäuse 10 der Aufbereitungskammer 8 ist hierzu zum Erzeugen von magnetischen Wechselfeldern ausgebildet. Energie wird hierdurch bevorzugt in Form eines elektromagnetischen Wechselfeldes auf die aufzubereitenden medizinischen, insbesondere metallischen, Instrumente übertragen und dort in Wärme umgewandelt.

Gemäß einem alternativen Ausführungsbeispiel können die in Figur 2 gezeigten Bestandteile des Sterilisators 2, insbesondere die Steuervorrichtung 25, die Energieversorgung 28, die Medienversorgung 33 und die Medienzuführung 24 in der Tür 6 des Sterilisators 1 angeordnet sein. Zusammen mit einem Teil des Gehäuses 10 der Aufbereitungskammer 8, welches die zumindest eine erste Leiterbahn 13 aufweist, bildet die Tür somit ein modulares Bauteil, welches es ermöglicht, dieses für eine Vielzahl von unterschiedlich ausgebildeten Aufbereitungsvorrichtungen zu verwenden.

Figur 2A zeigt einen ersten Ausschnitt des Gehäuses 10 der Aufbereitungskammer 8 aus Figur 2. Um die zumindest eine Leiterbahn 13 direkt auf der Kammer 8, insbesondere an deren Außenseite 12 elektrisch isoliert zu positionieren, wird erfindungsgemäß auf der Kammer 8 eine Trägerschicht 14 aus dielektrischem Material aufgebracht. Die Deckschicht 15, welche ebenfalls ein dielektrisches Material aufweist, dient zum elektrischen Isolieren der zumindest einen Leiterbahn 13 gegenüber elektrisch leitender Bauteile in der Aufbereitungsvorrichtung 1, welche insbesondere benachbart zur der Aufbereitungskammer 8 in der Vorrichtung 1 angeordnet sind.

Die zumindest zwei elektrischen Kontakte 19 und 20, welche zur Versorgung der zumindest einen Leiterbahn 13 mit elektrischer Energie dienen, sind bevorzugt auf der Trägerschicht 14 oder direkt auf der Leiterbahn 13 positioniert und stehen mit dieser in elektrischer Verbindung. Zur elektrischen Isolierung der Kontakte 19 und 20 sind diese bevorzugt ebenfalls von der Deckschicht 15 umgeben bzw. überzogen. Die elektrischen Zuführungsleitungen 29 und 30, welche von einer eigenen Isolierung umhüllt sind, erstrecken sich hierbei durch die Deckschicht 15 zu den elektrischen Kontakten 19 und 20.

Durch diese Anordnung der Leiterbahn 13 direkt auf der Kammer 8 ist es möglich Wärmeenergie direkt an der Aufbereitungskammer 8, insbesondere an dessen Außenseite 12 zu erzeugen. Die erzeugt Wärme diffundiert durch das Gehäuse 10 und strahlt von der Innenseite 11 des Gehäuses 10 in die Kammer 8.

Figur 2B zeigt einen zweiten Ausschnitt des Gehäuses 10 der Aufbereitungskammer 8 aus Figur 2. Hierbei ist die zweite Leiterbahn 18 an der Innenseite 11 des Gehäuses 10 der Aufbereitungskammer 8 angeordnet und zur Erwärmung bzw. Verdampfung eines Betriebsmediums bevorzugt spiralförmig ausgebildet. Insbesondere zeigt Figur 2B einen Schnitt durch die spiralförmige Leiterbahn 18 und somit mehrere Abschnitte 16, 17 der Leiterbahn 18. Diese Abschnitte 16, 17 sind bevorzugt in unterschiedlichen Abstanden A und B zueinander angeordnet. So weist bei diesem Ausführungsbeispiel die Leiterbahn 18 in einem zentralen Bereich der Spirale kleinere Abstande A zwischen den Abschnitten 16 und 17 der Leiterbahn 18 auf, als in einem äußeren Bereich der Leiterbahn 18. Der Abstand B zwischen den Abständen 16, 17 im äußeren Umfang der Spirale ist somit größer. Hierdurch ist es möglich die erzeugte Wärmemenge pro Fläche an dem Gehäuse 10 zu variieren.

Auch bei diesem Ausführungsbeispiel ist die Leiterbahn 18 auf einer Trägerschicht 14A angeordnet, um die Leiterbahn 18 elektrisch isoliert auf der Innenseite 11 des Gehäuses 10 zu positionieren. Um nun die Leiterbahn 18 gegenüber dem zumindest einen aufzubereitenden medizinischen Instrument elektrischen zu isolieren, ist diese ebenfalls mit einer Deckschicht 15A überzogen. Diese Schicht 15A umgibt die Leiterbahn 18, insbesondere die mehreren Abschnitte 16, 17, jeweils an bevorzugt drei Seiten, insbesondere an deren Oberseite sowie an deren seitlichen Flächen.

Zur Versorgung der Leiterbahnen 18 mittels elektrischer Zuführungsleitungen 31 und 32 mit elektrischer Energie weist diese zumindest zwei elektrische Kontakte 19A und 20A auf. Diese sind ebenfalls bevorzugt auf der Trägerschicht 14A oder direkt auf der Leiterbahn 18 positioniert und stehen mit dieser in elektrischer Verbindung. Die Kontaktierung der Leiterbahn 18 erfolgt bevorzugt von der Außenseite 12 des Gehäuse 10 der Kammer 8. Hierzu erstrecken sich die Kontakte 19A und 20A durch das Gehäuse 10. Um die Kontakte 19A und 20A gegenüber dem elektrisch leitendem Gehäuse 10 zu isolieren, sind die Kontakte 19A und 20A jeweils von einer Isolierung 21 und 22 umgeben. Die Isolierungen 21 und 22 sind bevorzugt zylindrisch ausgebildet und weisen eine zentrale Bohrung zur Aufnahme der Kontakte 19A und 20A auf.

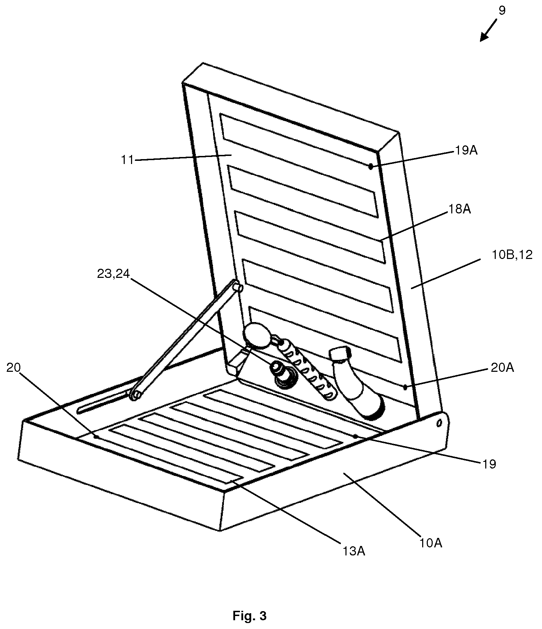

In Figur 3 ist eine perspektivische Darstellung eines Aufbereitungsträgers 9 zur Aufnahme von zumindest einem medizinischen, insbesondere zahnärztlichen, Instrument in einer Aufbereitungsvorrichtung 1 abgebildet. Hierbei ist die Aufbereitungsvorrichtung 1 bevorzugt zum Reinigen mittels einer Flüssigkeit, zum Sterilisieren mittels gesättigtem Wasserdampf und zum Pflegen mittels zumindest eines Schmiermittels der medizinischen Instrumente ausgebildet. Der Aufbereitungsträger 9 selbst ist durch ein erstes und zweites Gehäuseteil 10A und 10B gebildet, welche zum Einbringen und Entnehmen des zumindest einen medizinischen, insbesondere zahnärztlichen, Instruments, relativ zueinander drehbar miteinander verbunden sind. Zwischen beiden Gehäuseteile 10A und 10B sind bevorzugt mehrere Anschlussvorrichtungen 23 zur Aufnahme der aufzubereitenden medizinischen Instrumente angeordnet. Die Anschlussvorrichtungen 23 weisen hierzu eine gemeinsame Basis auf, welche in diesem Ausführungsbeispiel bevorzugt drehbar gelagert ist. Die Basis bildet hierbei ein drittes Gehäuseteil, insbesondere die Rückseite des Aufbereitungsträgers 9. Die Anschlussvorrichtungen 23 sind bei diesem Ausführungsbeispiel gleichzeitig als Medienzuführung 24 ausgeführt. Hierzu sind diese von zumindest einer Bohrung, die eine Quelle eines Aufbereitungsmediums mit dem Inneren der aufgesteckten Instrumente verbindet, durchsetzt. Bevorzugt sind in den Anschlüssen 23 mehrere Bohrungen vorgesehen, so dass die Instrumente mit mehreren Medien aufbereitet werden können. Die Bohrungen erstrecken sich bevorzugt durch die gemeinsame Basis der Anschlüsse 23. Wird der Aufbereitungsträger 9 in eine Aufbereitungsvorrichtung 1 eingesetzt, so werden die Bohrungen über eine Kupplungsvorrichtung mit den Quellen der Medien verbunden. Bevorzugt weist das Gehäuse 10A oder 10B zumindest eine weitere Medienzuführung zum Einbringen zumindest eines Betriebsmediums in den Aufbereitungsträger 9 auf, welche insbesondere in Form einer oder mehrerer Düsen ausgebildet ist. Diese Düsen dienen bevorzugt der Außenreinigung der medizinischen Instrumente.

Gemäß der Erfindung weisen die Gehäuseteile 10A und 10B des Aufbereitungsträgers 9 des Weiteren jeweils zumindest eine Leiterbahn 13A und 18A aus elektrisch leitendem Material zum Umwandeln elektrischer Energie in Wärmeenergie auf. Die Leiterbahnen 13A und 18A sind hierbei bevorzugt an der Innenseite 11 der beiden Gehäuseteile 10A, 10B angeordnet und bevorzugt stufenförmig ausgebildet. Zur Versorgung beider Leiterbahnen 13A und 18A mit elektrischer Energie umfassen diese jeweils zumindest zwei elektrische Kontakte 19, 20 und 19A, 20A. Durch die getrennte Kontaktierung beider Leiterbahnen 13A und 13B sind diese separat mit elektrischer Energie versorgbar. Die Kontaktierung der Leiterbahnen 13A und 13B, insbesondere der elektrischen Kontakte 19, 20 und 19A, 20A, erfolgt bevorzugt von der Außenseite 12 der Gehäuseteile 10A und 10B. Hierzu erstrecken sich die Kontakte 19, 20 und 19A, 20A bevorzugt durch die Gehäuseteile 10A und 10B. Sind die Gehäuseteile 10A und 10B aus einem elektrisch leitendem Material gefertigt, sind die Kontakte 19, 20 und 19A, 20A, wie bereits in Figur 2B gezeigt, von einer Isolierung 21, 22 umgeben.

Des Weiteren weisen die Leiterbahnen 13A und 18A jeweils mehrere Abschnitte auf, welche in unterschiedlichen Abständen zueinander an dem Gehäuse 10A, 10B des Aufbereitungsträgers 9 angeordnet sind. Dies sowie die separate Versorgung der Leiterbahnen 13A und 18A mit elektrischer Energie ermöglicht die erzeugte Wärmemenge pro Fläche an dem Gehäuse 10A, 10B zu variieren, wodurch eine homogene Temperaturverteilung in dem Träger 9 erzielt werden kann. Hierzu weisen die mehreren Abschnitte der Leiterbahn 13A in der unteren Gehäusehälfte 10A bevorzugt kleinere Abstände auf, als die Leiterbahn 18A in der oberen Hälfte 10B.

Auch bei diesem Ausführungsbeispiel des Aufbereitungsträgers 9 weisen die Leiterbahnen 13A und 18A eine Trägerschicht und/ oder Deckschicht aus dielektrischem Material auf, um die Leiterbahn 13A und 18A elektrisch zu isolieren. Die Deck- und Trägerschicht sowie die elektrische Leiterbahnen 13A und 13B sind auch in diesem Ausführungsbeispiel als Dünnschicht oder Dickschicht ausgebildet.

## Patentansprüche

1. Aufbereitungskammer (8) oder Aufbereitungsträger (9) zur Verwendung in einer Vorrichtung (1) zur Aufbereitung von zumindest einem medizinischen, insbesondere zahnärztlichen, Instrument mit einem Gehäuse (10, 10A, 10B), welches das zumindest eine medizinische Instrument zumindest teilweise umgibt, wobei das Gehäuse (10, 10A, 10B) zumindest eine Leiterbahn (13, 13A, 18, 18A) aus elektrisch leitendem Material zum Umwandeln elektrischer Energie in Wärmeenergie und zumindest zwei elektrische Kontakte (19, 20, 19A, 20A) aufweist, um die zumindest eine Leiterbahn (13, 13A, 18, 18A) mit elektrischer Energie zu versorgen, **gekennzeichnet durch** eine direkt auf der Aufbereitungskammer (8) oder dem Träger (9) aufgebrachte, als Beschichtung, insbesondere als Dünnschicht oder Dickschicht, ausgebildete, elektrische Leiterbahn (13, 13A, 18, 18A).

2. Aufbereitungskammer (8) oder Aufbereitungsträger (9) nach Anspruch 1, **dadurch gekennzeichnet, dass** die zumindest eine Leiterbahn (13, 13A, 18, 18A) eine Trägerschicht (14, 14A) und/ oder Deckschicht (15, 15A) aus dielektrischem Material aufweist, um die Leiterbahn (13, 13A, 18, 18A) elektrisch zu isolieren.

3. Aufbereitungskammer (8) oder Aufbereitungsträger (9) nach Anspruch 1 oder 2, **dadurch gekennzeichnet, dass** die zumindest eine Leiterbahn (13, 13A, 18, 18A) mehrere Abschnitte (16, 17) aufweist, welche in unterschiedlichen Abständen (A, B) zueinander an dem Gehäuse (10, 10A, 10B) der Aufbereitungskammer (8) oder des Aufbereitungsträgers (9) angeordnet sind.

4. Aufbereitungskammer (8) oder Aufbereitungsträger (9) nach einem der vorherigen Ansprüche, **dadurch gekennzeichnet, dass** die zumindest eine Leiterbahn (13, 13A, 18, 18A) an der Innenseite (11) und/ oder Außenseite (12) des Gehäuses (10, 10A, 10B) angeordnet ist.

5. Aufbereitungskammer (8) oder Aufbereitungsträger (9) nach einem der vorherigen Ansprüche, **dadurch gekennzeichnet, dass** die zumindest eine Leiterbahn (13, 13A, 18, 18A) derart an dem Gehäuse (10, 10A, 10B) angeordnet ist, dass diese das zumindest eine medizinische Instrument zumindest an zwei Seiten umgibt.

6. Aufbereitungskammer (8) oder Aufbereitungsträger (9) nach einem der vorherigen Ansprüche, **dadurch gekennzeichnet, dass** das Gehäuse (10, 10A, 10B) zumindest zwei Leiterbahnen (13, 13A, 18, 18A) aufweist, welche separat mit elektrischer Energie versorgbar sind.

7. Aufbereitungskammer (8) oder Aufbereitungsträger (9) nach Anspruch 2, **dadurch gekennzeichnet, dass** die Trägerschicht (14, 14A) und/ oder die Deckschicht (15, 15A) durch eine Beschichtung, insbesondere durch eine Dünnschicht oder Dickschicht, gebildet sind.

8. Aufbereitungskammer (8) oder Aufbereitungsträger (9) nach einem der vorherigen Ansprüche, **dadurch gekennzeichnet, dass** das Gehäuse für das zumindest eine medizinische Instrument zumindest ein erstes und zweites Gehäuseteil (10A, 10B) aufweist, welche zum Einbringen und Entnehmen des zumindest einen medizinischen, insbesondere zahnärztlichen, Instruments, relativ zueinander drehbar oder schiebbar miteinander verbunden sind.

9. Aufbereitungskammer (8) oder Aufbereitungsträger (9) nach einem der vorherigen Ansprüche, **dadurch gekennzeichnet, dass** das Gehäuse (10, 10A, 10B) zumindest eine Anschlussvorrichtung (23) zur Aufnahme des zumindest einen medizinischen Instruments aufweist.

10. Aufbereitungskammer (8) oder Aufbereitungsträger (9) nach einem der vorherigen Ansprüche, **dadurch gekennzeichnet, dass** das Gehäuse (10, 10A, 10B) zumindest eine Medienzuführung (24) zum Einbringen zumindest eines Betriebsmediums in die Aufbereitungskammer (8) oder in den Aufbereitungsträger (9) aufweist.

11. Vorrichtung (1) zur Aufbereitung von zumindest einem medizinischen, insbesondere zahnärztlichen, Instrument, aufweisend eine Steuervorrichtung (25) zum Steuern und/ oder Regeln eines Aufbereitungsprozesses, eine Energieversorgung (28) zum Anschluss der Aufbereitungsvorrichtung (1) an eine Energiequelle, zumindest eine Medienversorgung (33) mit zumindest einem Medienspeicher und/ oder zumindest einem Anschluss für eine externe Medienquelle sowie eine Aufbereitungskammer (8) und/ oder einen Aufbereitungsträger (9) für das zumindest eine medizinische, insbesondere zahnärztliche, Instrument, **dadurch gekennzeichnet, dass** die Aufbereitungskammer (8) und/ oder der Aufbereitungsträger (9) nach einem der Ansprüche 1 bis 10 ausgebildet sind.

12. Vorrichtung (1) nach Anspruch 11, **dadurch gekennzeichnet, dass** das die Aufbereitungskammer (8) und/ oder der Aufbereitungsträger (9) lösbar von der Aufbereitungsvorrichtung (1) ausgebildet sind.

13. Vorrichtung (1) nach einem der Ansprüche 11 oder 12, **dadurch gekennzeichnet, dass** die Aufbereitungsvorrichtung (1) eine Tür (6) aufweist, wobei die Steuervorrichtung (25), die Energieversorgung (28) oder die Medienversorgung (33) und zumindest eines der beiden Gehäuseteile (10A, 10B) der Aufbereitungskammer (8) oder des Aufbereitungsträgers (9) in oder an der Tür (6) der Aufbereitungskammer (8) angeordnet sind.

14. Vorrichtung (1) nach einem der Ansprüche 11 bis 13, **dadurch gekennzeichnet, dass** die Steuervorrichtung (25) eine Schaltung (26) zur Identifikation des zumindest einen in der Aufbereitungskammer (8) oder in dem Aufbereitungsträger (9) aufgenommenen Instruments aufweist und die zumindest eine Leiterbahn (13, 13A, 18, 18A) an dem Gehäuse (10, 10A, 10B) der Aufbereitungskammer (8) oder an dem Aufbereitungsträger (9) zum Senden und/ oder Empfangen von Daten und/ oder Energie ausgebildet ist.

15. Vorrichtung (1) nach einem der Ansprüche 11 bis 14, **dadurch gekennzeichnet, dass** die Steuervorrichtung (25) einen Schaltkreis (27) zur Erzeugung von Wirbelströmen in der Aufbereitungskammer (8) oder in dem Aufbereitungsträger (9) aufweist und die zumindest eine Leiterbahn (13, 13A, 18, 18A) an dem Gehäuse (10, 10A, 10B) der Aufbereitungskammer (8) oder an dem Aufbereitungsträger (9) zum Erzeugen von magnetischen Wechselfeldern ausgebildet ist.

## Claims

1. Treatment chamber (8) or treatment carrier (9) for use in a device (1) for treating at least one medical, in particular dental, instrument having a housing (10, 10A, 10B) which at least partially surrounds the at least one medical instrument, wherein the housing (10, 10A, 10B) comprises at least one conductor (13, 13A, 18, 18A) made of an electrically conductive material for converting electrical energy into thermal energy, and at least two electrical contacts (19, 20, 19A, 20A) to supply electrical energy to the at least one conductor (13, 13A, 18, 18A), **characterized by** an electric conductor (13, 13A, 18, 18A) which is applied directly to the treatment chamber (8) or the carrier (9) and which is formed by a coating, in particular by a thin film or a thick film.

2. Treatment chamber (8) or treatment carrier (9) according to claim 1, **characterized in that** the at least one conductor (13, 13A, 18, 18A) comprises a carrier layer (14, 14A) and/ or cover layer (15, 15A) made of a dielectric material to electrically insulate the conductor (13, 13A, 18, 18A).

3. Treatment chamber (8) or treatment carrier (9) according to claim 1 or 2, **characterized in that** the at least one conductor (13, 13A, 18, 18A) comprises a plurality of sections (16, 17), which are arranged at different distances (A, B) from one another on the housing (10, 10A, 10B) of the treatment chamber (8) or of the treatment carrier (9).

4. Treatment chamber (8) or treatment carrier (9) according to any one of the preceding claims, **characterized in that** the at least one conductor (13, 13A, 18, 18A) is arranged on the inside (11) and/ or the outside (12) of the housing (10, 10A, 10B).

5. The treatment chamber (8) or treatment carrier (9) according to any one of the preceding claims, **characterized in that** the at least one conductor (13, 13A, 18, 18A) is arranged on the housing (10, 10A, 10B) in such a way that it surrounds the at least one medical instrument on at least two sides.

6. Treatment chamber (8) or treatment carrier (9) according to any one of the preceding claims, **characterized in that** the housing (10, 10A, 10B) comprises at least two conductors (13, 13A, 18, 18A) which can be supplied separately with electrical energy.

7. Treatment chamber (8) or treatment carrier (9) according to claim 2, **characterized in that** the carrier layer (14, 14A) and/ or the cover layer (15, 15A) is/are formed by a coating, in particular by a thin-film or thick-film.

8. Treatment chamber (8) or treatment carrier (9) according to any one of the preceding claims, **characterized in that** the housing for the at least one medical instrument comprises at least one first and second housing part (10A, 10B) which are connected rotatably or displaceably relative to one another for introducing and removing the at least one medical, in particular dental, instrument.

9. Treatment chamber (8) or treatment carrier (9) according to any one of the preceding claims, **characterized in that** the housing (10, 10A, 10B) comprises at least one connecting device (23) for receiving the at least one medical instrument.

10. Treatment chamber (8) or treatment carrier (9) according to any one of the preceding claims, **characterized in that** the housing (10, 10A, 10B) comprises at least one media feed (24) for introducing at least one operating medium into the treatment chamber (8) or into the treatment carrier (9).

11. Device (1) for treating at least one medical, in particular dental, instrument, comprising a control unit (25) for controlling and/ or regulating a treatment process, a power supply (28) for connecting the treatment device (1) to a power source, at least one media supply (33) having at least one media storage device and/ or at least one connection for an external media source as well as a treatment chamber (8) and/ or a treatment carrier (9) for the least one medical, in particular dental, instrument, **characterized in that** the treatment chamber (8) and/ or the treatment carrier (9) is/ are designed according to any one of claims 1 to 10.

12. Device (1) according to claim 11, **characterized in that** the treatment chamber (8) and/ or the treatment carrier (9) is/ are designed to be detachable from the treatment device (1).

13. Device (1) according to any one of claims 11 or 12, **characterized in that** the treatment device (1) comprises a door (6), wherein the control unit (25), the power supply (28) or the media supply (33) and at least one of the two housing parts (10A, 10B) of the treatment chamber (8) or of the treatment carrier (9) is arranged in or on the door (6) of the treatment chamber (8).

14. Device (1) according to any one of claims 11 to 13, **characterized in that** the control unit (25) comprises a circuit (26) for identification of the at least one instrument accommodated in the treatment chamber (8) or in the treatment carrier (9) and the at least one conductor (13, 13A, 18, 18A) is formed on the housing (10, 10A, 10B) of the treatment chamber (8) or on the treatment carrier (9) for sending and/ or receiving data and/ or energy.

15. Device (1) according to any one of claims 11 to 14, **characterized in that** the control unit (25) comprises a circuit (27) for generating eddy currents in the treatment chamber (8) or in the treatment carrier (9) and the at least one conductor (13, 13A, 18, 18A) is designed on the housing (10, 10A, 10B) of the treatment chamber (8) or on the treatment carrier (9) for generating alternating magnetic fields.

## Revendications

1. Chambre de traitement (8) ou support de traitement (9) pour l'utilisation dans un dispositif (1) pour traiter au moins un instrument médical, en particulier dentaire, ayant un boîtier (10, 10A, 10B) qui entoure au moins partiellement l'au moins un instrument médical, dans lequel le boîtier (10, 10A, 10B) comprend au moins un conducteur (13, 13A, 18, 18A) formé en un matériau électroconducteur pour convertir de l'énergie électrique en énergie thermique, et au moins deux contacts électriques (19, 20, 19A, 20A) pour alimenter en énergie électrique l'au moins un conducteur (13, 13A, 18, 18A), **caractérisé(e) par** un conducteur électrique (13, 13A, 18, 18A) qui est directement appliqué à la chambre de traitement (8) ou au support de traitement (9) et qui est formé par un revêtement, en particulier par une pellicule mince ou une pellicule épaisse.

2. Chambre de traitement (8) ou support de traitement (9) selon la revendication 1, **caractérisé(e) en ce que** l'au moins un conducteur (13, 13A, 18, 18A) comprend une couche de support (14, 14A) et/ou une couche de recouvrement (15, 15A) formée en un matériau diélectrique pour isoler électriquement le conducteur (13, 13A, 18, 18A).

3. Chambre de traitement (8) ou support de traitement (9) selon la revendication 1 ou 2, **caractérisé(e) en ce que** l'au moins un conducteur (13, 13A, 18, 18A) comprend une pluralité de sections (16, 17) qui sont disposées à différentes distances (A, B) les unes des autres sur le boîtier (10, 10A, 10B) de la chambre de traitement (8) ou du support de traitement (9).

4. Chambre de traitement (8) ou support de traitement (9) selon l'une quelconque des revendications précédentes, **caractérisé(e) en ce que** l'au moins un conducteur (13, 13A, 18, 18A) est disposé à l'intérieur (11) et/ou à l'extérieur (12) du boîtier (10, 10A, 10B).

5. Chambre de traitement (8) ou support de traitement (9) selon l'une quelconque des revendications précédentes, **caractérisé(e) en ce que** l'au moins un conducteur (13, 13A, 18, 18A) est disposé sur le boîtier (10, 10A, 10B) d'une manière à ce qu'il entoure l'au moins un instrument médical sur au moins deux côtés.

6. Chambre de traitement (8) ou support de traitement (9) selon l'une quelconque des revendications précédentes, **caractérisé(e) en ce que** le boîtier (10, 10A, 10B) comprend au moins deux conducteurs (13, 13A, 18, 18A) qui peuvent être alimentés séparément avec de l'énergie électrique.

7. Chambre de traitement (8) ou support de traitement (9) selon la revendication 2, **caractérisé(e) en ce que** la couche de support (14, 14A) et/ou la couche de recouvrement (15, 15A) est/sont formée(s) par un revêtement, en particulier par une pellicule mince ou une pellicule épaisse.

8. Chambre de traitement (8) ou support de traitement (9) selon l'une quelconque des revendications précédentes, **caractérisé(e) en ce que** le boîtier pour l'au moins un instrument médical comprend au moins une première et une deuxième partie de boîtier (10A, 10B), lesquelles sont raccordées de manière rotative ou déplaçable l'une par rapport à l'autre pour l'introduction et le retrait de l'au moins un instrument médical, en particulier dentaire.

9. Chambre de traitement (8) ou support de traitement (9) selon l'une quelconque des revendications précédentes, **caractérisé(e) en ce que** le boîtier (10, 10A, 10B) comprend au moins un dispositif de raccordement (23) pour recevoir l'au moins un instrument médical.

10. Chambre de traitement (8) ou support de traitement (9) selon l'une quelconque des revendications précédentes, **caractérisé(e) en ce que** le boîtier (10, 10A, 10B) comprend au moins une alimentation de fluides (24) pour introduire au moins un fluide de fonctionnement dans la chambre de traitement (8) ou dans le support de traitement (9).

11. Dispositif (1) pour traiter au moins un instrument médical, en particulier dentaire, comprenant une unité de contrôle (25) pour contrôler et/ou réguler un processus de traitement, une alimentation énergétique (28) pour raccorder le dispositif de traitement (1) à une source d'énergie, au moins une alimentation de fluides (33) ayant au moins un dispositif de stockage de fluides et/ou au moins un raccord pour une source de fluides externe ainsi qu'une chambre de traitement (8) et/ou un support de traitement (9) pour l'au moins un instrument médical, en particulier dentaire, **caractérisé en ce que** la chambre de traitement (8) et/ou le support de traitement (9) est/sont réalisés selon l'une quelconque des revendications 1 à 10.

12. Dispositif (1) selon la revendication 11, **caractérisé en ce que** la chambre de traitement (8) et/ou le support de traitement (9) est/sont réalisés pour pouvoir être détachés du dispositif de traitement (1).

13. Dispositif (1) selon l'une quelconque des revendications 11 ou 12, **caractérisé en ce que** le dispositif de traitement (1) comprend une porte (6), dans lequel l'unité de contrôle (25), l'alimentation énergétique (28) ou l'alimentation de fluides (33) et au moins l'une des deux parties de boîtier (10A, 10B) de la chambre de traitement (8) ou du support de traitement (9) sont disposés dans la porte (6), ou sur celle-ci, de la chambre de traitement (8).

14. Dispositif (1) selon l'une quelconque des revendications 11 à 13, **caractérisé en ce que** l'unité de contrôle (25) comprend un circuit (26) pour l'identification de l'au moins un instrument logé dans la chambre de traitement (8) ou dans le support de traitement (9), et l'au moins un conducteur (13, 13A, 18, 18A) étant formé sur le boîtier (10, 10A, 10B) de la chambre de traitement (8) ou sur le support de traitement (9) pour envoyer et/ou recevoir des données et/ou de l'énergie.

15. Dispositif (1) selon l'une quelconque des revendications 11 à 14, **caractérisé en ce que** l'unité de contrôle (25) comprend un circuit (27) pour générer des courants de Foucault dans la chambre de traitement (8) ou dans le support de traitement (9), et l'au moins un conducteur (13, 13A, 18, 18A) sur le boîtier (10, 10A, 10B) de la chambre de traitement (8) ou du support de traitement (9) étant réalisé pour générer des champs magnétiques alternatifs.
